# FASCICULE DE BREVET EUROPEEN

(11) **EP 3 498 231 B1**
(45) Date de publication et mention de la délivrance du brevet: **18.11.2020**
(21) Numéro de dépôt: 18208954.0
(22) Date de dépôt: 28.11.2018
(51) Int. Cl.: A61F 2/38

(54) **GAMME D'IMPLANTS FÉMORAUX ET PROTHÈSE TOTALE DE GENOU COMPRENANT UN IMPLANT FÉMORAL SÉLECTIONNÉ DANS UNE TELLE GAMME D'IMPLANTS FÉMORAUX**
FEMORALIMPLANTATSORTIMENT UND KNIE-TOTALPROTHESE, DIE EIN AUSGEWÄHLTES FEMORALIMPLANTAT AUS DIESEM FEMORALIMPLANTATSORTIMENT UMFASST
RANGE OF FEMORAL IMPLANTS AND FULL KNEE PROSTHESIS COMPRISING A FEMORAL IMPLANT SELECTED FROM SUCH A RANGE OF FEMORAL IMPLANTS

(30) Priorité: 29.11.2017 FR 1761369
(43) Date de publication de la demande: 19.06.2019
(73) Titulaire: Amplitude, 26000 Valence (FR); SCORE 2, 69003 Lyon (FR)
(72) Inventeur: CHAVANE, Hervé, 69300 Caluire et Cuire (FR); ROLLIER, Jean-Charles, 74370 Saint Martin Bellevue (FR); HAUCK, Werner, Landstuhl (DE); JACQUOT, Laurent, 74370 Charvonnex (FR); CHEVILLOTTE, Christophe Jean François Marie, 71640 Givry (FR); DELALANDE, Jean-Luc Christian Jules, 45560 Saint-Denis-En-Val (FR); DENJEAN, Stéphane, 71960 La Roche Vineuse (FR); GAILLARD, Thierry Alain Michel, 69003 Lyon (FR); TAYOT, Olivier Louis, 69300 Caluire-et-Cuire (FR); CHATAIN, Frédéric Georges Marie, 38190 Bernin (FR); CHARLIER, Hervé, 14763 Glabais (BE); VANCABEKE, Michel, 1410 Waterloo (BE); REGENET, Jérémy, 26300 Besayes (FR)
(74) Mandataire: Chevalier, Renaud Philippe

(56) Documents cités:
- FR-A1- 2 955 482
- GB-A- 2 296 443
- US-A1- 2014 142 713

## Description

La présente invention concerne une gamme d'implants fémoraux destinés chacun à être implanté sur une extrémité réséquée d'un fémur de façon à coopérer avec un insert tibial. De plus, la présente invention concerne une prothèse totale de genou comprenant un implant fémoral sélectionné sélectionné dans une telle gamme d'implants fémoraux.

La présente invention trouve application dans le domaine de la chirurgie orthopédique du genou pour fabriquer une gamme d'implants fémoraux et une prothèse totale de genou.

Il est connu de l'état de la technique FR 2 838 634 A1 de prévoir une gamme d'implants fémoraux de l'art antérieur. Dans l'art antérieur, chaque implant fémoral appartenant à une gamme d'implants fémoraux comprend i) une portion de réception pour recevoir l'extrémité réséquée du fémur, et ii) une portion de coopération pour coopérer avec l'insert tibial. La portion de coopération présente une surface antérieure et une surface postérieure. La surface antérieure et la surface postérieure sont séparées sensiblement par un plan frontal incluant l'axe de révolution de condyles postérieurs. La surface antérieure de chaque implant fémoral présente un bord médial et un bord latéral délimitant des largeurs médio-latérales mesurées parallèlement à l'axe de révolution de condyles postérieurs.

L'état de la technique peut également être illustré par l'enseignement du document US2014/142713 qui divulgue une gamme d'implants fémoraux selon le préambule de la revendication 1.

Lorsqu'un chirurgien sélectionne, dans une gamme d'implants fémoraux de l'art antérieur, un implant fémoral pour composer une prothèse de genou, le chirurgien cherche à obtenir la compatibilité entre, d'une part, la morphométrie du patient et, d'autre part, soit la largeur médio-latérale soit la longueur antéro-postérieure de l'implant fémoral à implanter.

Cependant, la sélection d'un implant fémoral dans une gamme d'implants fémoraux de l'art antérieur s'avère donc parfois difficile à optimiser.

Dans les cas où le chirurgien est conduit à privilégier la compatibilité de la morphométrie avec la dimension antéro-postérieure de l'implant fémoral, il existe parfois un risque de fracture de la métaphyse distale du fémur au-dessus de l'implant fémoral, car la mise en place d'un implant fémoral trop petit suivant sa dimension antéro-postérieure peut conduire à réaliser une encoche (opération parfois dénommé sous le terme anglais « *notching* ») dans l'os cortical antérieur.

Inversement, dans les cas où le chirurgien est conduit à privilégier la compatibilité de la morphométrie avec la largeur médio-latérale de l'implant fémoral, il existe parfois un risque que l'implant fémoral dépasse sur le côté latéral ou sur le côté médial. En effet, le bord médial et/ou le bord latéral de l'implant fémoral touche l'aileron rotulien médial et/ou latéral, ce qui peut induire une douleur chez le patient équipé de la prothèse.

La présente invention vise notamment à résoudre, en tout ou partie, les problèmes mentionnés ci-avant.

À cet effet, l'invention a pour objet une gamme d'implants fémoraux, comprenant plusieurs implants fémoraux destinés chacun à être implanté sur une extrémité réséquée d'un fémur, chaque implant fémoral comprenant :
i) une portion de réception définissant une cavité adaptée pour recevoir l'extrémité réséquée du fémur, et
ii) une portion de coopération agencée pour coopérer avec un insert tibial, la portion de coopération présentant une surface antérieure et une surface postérieure, la surface antérieure et la surface postérieure étant séparées sensiblement par un plan frontal incluant un axe de révolution de condyles postérieurs autour duquel tournent les condyles postérieurs lorsque l'implant fémoral est en service,
la surface antérieure respective de chaque implant fémoral présentant un bord médial et un bord latéral délimitant des largeurs médio-latérales mesurées parallèlement à l'axe de révolution de condyles postérieurs,
où la gamme d'implants fémoraux comprend au moins sept implants fémoraux ordonnés par dimensions croissantes suivant des rangs allant de 1 à 7,
et une largeur médio-latérale primaire, mesurée dans le plan frontal incluant l'axe de révolution de condyles postérieurs, est égale :
- à 58,5 ± 1.5 mm pour l'implant fémoral de rang 1 ;
- à 61,5 ± 1.5 mm pour l'implant fémoral de rang 2 ;
- à 64,5 ± 1.5 mm pour l'implant fémoral de rang 3 ;
- à 67,5 ± 1.5 mm pour l'implant fémoral de rang 4 ;
- à 70,5 ± 1.5 mm pour l'implant fémoral de rang 5 ;
- à 73,5 ± 1.5 mm pour l'implant fémoral de rang 6 ;
- à 76,5 ± 1.5 mm pour l'implant fémoral de rang 7 ;
et où une telle gamme d'implants fémoraux est remarquable en ce que la surface antérieure respective de chaque implant fémoral présente une largeur médio-latérale secondaire, mesurée dans un plan secondaire qui inclut un axe proximal et qui forme un angle de 60 degrés avec le plan frontal incluant l'axe de révolution de condyles postérieurs en projection dans un plan sagittal, égale :
- à 45,0 ± 1 mm pour l'implant fémoral de rang 1 ;
- à 47,0 ± 1 mm pour l'implant fémoral de rang 2 ;
- à 49,0 ± 1 mm pour l'implant fémoral de rang 3 ;
- à 51,0 ± 1 mm pour l'implant fémoral de rang 4 ;
- à 53,0 ± 1 mm pour l'implant fémoral de rang 5 ;
- à 55,5 ± 1,5 mm pour l'implant fémoral de rang 6 ; et
- à 58,5 ± 1,5 mm pour l'implant fémoral de rang 7.

Ainsi, une telle gamme d'implants fémoraux permet de sélectionner un implant fémoral ayant une hauteur suffisante pour réduire les risques de fracture du fémur au-dessus de l'implant fémoral, tout en réduisant les risques que l'implant fémoral ne dépasse sur le côté latéral ou sur le côté médial. Par conséquent, une telle gamme d'implants fémoraux permet de conserver l'articulation fémoro-patellaire par les ailerons rotuliens interne et externe, tout en réduisant la largeur médio-latérale maximale de l'implant fémoral.

L'axe de révolution de condyles postérieurs correspond à l'axe central des cylindres circulaires les plus congruents avec la forme des condyles naturels du fémur.

De plus, avec de telles largeurs médio-latérale secondaires, cette gamme d'implants fémoraux permet d'éviter les risques de fracture du fémur au-dessus de l'implant fémoral, tout en évitant les risques que l'implant fémoral ne dépasse sur le côté latéral ou sur le côté médial.

Selon une variante, la largeur médio-latérale primaire, mesurée dans le plan frontal incluant l'axe de révolution de condyles postérieurs, est égale :
- à 58,1 mm pour l'implant fémoral de rang 1 ;
- à 60,5 mm pour l'implant fémoral de rang 2 ;
- à 63,1 mm pour l'implant fémoral de rang 3 ;
- à 66,0 mm pour l'implant fémoral de rang 4 ;
- à 69,1 mm pour l'implant fémoral de rang 5 ;
- à 72,4 mm pour l'implant fémoral de rang 6 ;
- à 76,0 mm pour l'implant fémoral de rang 7.

Selon un mode de réalisation, la gamme d'implants fémoraux peut comprendre au moins neuf implants fémoraux ordonnés par dimensions croissantes suivant des rangs allant de 0 à 8,
une largeur médio-latérale primaire, mesurée dans le plan frontal incluant l'axe de révolution de condyles postérieurs, est égale :
- à 55,5 ± 1.5 mm pour l'implant fémoral de rang 0 ; et
- à 79,5 ± 1.5 mm pour l'implant fémoral de rang 8.

Selon une variante, la largeur médio-latérale primaire, mesurée dans le plan frontal incluant l'axe de révolution de condyles postérieurs, est égale :
- à 56,0 mm pour l'implant fémoral de rang 0 ; et
- à 80,0 mm pour l'implant fémoral de rang 8.

Selon une variante, la largeur médio-latérale secondaire est égale :
- à 44,9 mm pour l'implant fémoral de rang 1 ;
- à 47,7 mm pour l'implant fémoral de rang 2 ;
- à 49,3 mm pour l'implant fémoral de rang 3 ;
- à 51,4 mm pour l'implant fémoral de rang 4 ;
- à 53,5 mm pour l'implant fémoral de rang 5 ;
- à 56,7 mm pour l'implant fémoral de rang 6 ; et
- à 59,1 mm pour l'implant fémoral de rang 7.

Selon une possibilité, la surface antérieure respective de chaque implant fémoral présente une largeur médio-latérale secondaire égale :
- à 43,2 ± 1 mm pour l'implant fémoral de rang 0 ; et
- à 61,5 ± 1,5 mm pour l'implant fémoral de rang 8.

Selon une variante, cette largeur médio-latérale secondaire est égale :
- à 43,2 mm pour l'implant fémoral de rang 0 ; et
- à 62,2 mm pour l'implant fémoral de rang 8.

Selon un mode de réalisation, la surface antérieure respective de chaque implant fémoral peut présenter une largeur médio-latérale tertiaire, mesurée dans un plan tertiaire qui inclut un axe proximal et qui forme un angle de 90 degrés avec le plan frontal incluant l'axe de révolution de condyles postérieurs en projection dans un plan sagittal, égale :
- à 37,0 ± 1 mm pour l'implant fémoral de rang 1 ;
- à 39,0 ± 1 mm pour l'implant fémoral de rang 2 ;
- à 41,0 ± 1 mm pour l'implant fémoral de rang 3 ;
- à 43,0 ± 1 mm pour l'implant fémoral de rang 4 ;
- à 45,0 ± 1 mm pour l'implant fémoral de rang 5 ;
- à 47,0 ± 1 mm pour l'implant fémoral de rang 6 ; et
- à 49,0 ± 1 mm pour l'implant fémoral de rang 7.

Ainsi, une telle gamme d'implants fémoraux permet d'éviter les risques de fracture du fémur au-dessus de l'implant fémoral, tout en évitant les risques que l'implant fémoral ne dépasse sur le côté latéral ou sur le côté médial.

Selon une variante, cette largeur médio-latérale est égale :
- à 37,1 mm pour l'implant fémoral de rang 1 ;
- à 38,7 mm pour l'implant fémoral de rang 2 ;
- à 40,6 mm pour l'implant fémoral de rang 3 ;
- à 42,6 mm pour l'implant fémoral de rang 4 ;
- à 44,5 mm pour l'implant fémoral de rang 5 ;
- à 47,1 mm pour l'implant fémoral de rang 6 ; et
- à 48,9 mm pour l'implant fémoral de rang 7.

Selon un mode de réalisation, la surface antérieure respective de chaque implant fémoral peut présenter une largeur médio-latérale tertiaire égale :
- à 35,0 ± 1 mm pour l'implant fémoral de rang 0 ; et
- à 51,0 ± 1 mm pour l'implant fémoral de rang 8.

Selon une variante, cette largeur médio-latérale est égale :
- à 35,7 mm pour l'implant fémoral de rang 0 ; et
- à 51,5 mm pour l'implant fémoral de rang 8

Selon un mode de réalisation, la surface antérieure respective de chaque implant fémoral peut présenter une gorge de trochlée, la gorge de trochlée ayant un fond présentant un rayon de courbure, mesuré dans le plan frontal incluant l'axe de révolution de condyles postérieurs, égal :
- à 11,5 ± 0,5 mm pour l'implant fémoral de rang 1 ;
- à 11,5 ± 0,5 mm pour l'implant fémoral de rang 2 ;
- à 12,5 ± 0,5 mm pour l'implant fémoral de rang 3 ;
- à 13,5 ± 0,5 mm pour l'implant fémoral de rang 4 ;
- à 13,5 ± 0,5 mm pour l'implant fémoral de rang 5 ;
- à 14,5 ± 0,5 mm pour l'implant fémoral de rang 6 ; et
- à 14,5 ± 0,5 mm pour l'implant fémoral de rang 7.

Selon une variante, le rayon de courbure de la gorge de trochlée est égal :
- à 11,2 mm pour l'implant fémoral de rang 1 ;
- à 11,8 mm pour l'implant fémoral de rang 2 ;
- à 12,4 mm pour l'implant fémoral de rang 3 ;
- à 13,1 mm pour l'implant fémoral de rang 4 ;
- à 13,7 mm pour l'implant fémoral de rang 5 ;
- à 14,3 mm pour l'implant fémoral de rang 6 ; et
- à 14,9 mm pour l'implant fémoral de rang 7.

Selon un mode de réalisation, le fond de la gorge de trochlée présente un rayon de courbure égal :
- à 10,5 ± 0,5 mm pour l'implant fémoral de rang 0 ; et
- à 15,5 ± 0,5 mm pour l'implant fémoral de rang 8.

Selon une variante, le rayon de courbure de la gorge de trochlée est égal :
- à 10,8 mm pour l'implant fémoral de rang 0 ; et
- à 15,7 mm pour l'implant fémoral de rang 8.

Selon un mode de réalisation, la gorge de trochlée peut former, avec un plan sagittal et en projection dans un plan frontal, un angle égal à 5 degrés ± 1 degré pour chaque implant fémoral de la gamme.

Selon une possibilité, la gorge de trochlée forme, avec le plan sagittal et en projection dans le plan frontal, un angle égal :
- à 5,5 ± 0,5 degrés pour l'implant fémoral de rang 1 ;
- à 5,4 ± 0,5 degrés pour l'implant fémoral de rang 2 ;
- à 5,3 ± 0,5 degrés pour l'implant fémoral de rang 3 ;
- à 5,2 ± 0,5 degrés pour l'implant fémoral de rang 4 ;
- à 5,1 ± 0,5 degrés pour l'implant fémoral de rang 5 ;
- à 5,0 ± 0,5 degrés pour l'implant fémoral de rang 6 ; et
- à 4,9 ± 0,5 degrés pour l'implant fémoral de rang 7

Selon une autre possibilité, la gorge de trochlée forme, avec le plan sagittal et en projection dans le plan frontal, un angle égal :
- à 5,6 ± 0,5 degrés pour l'implant fémoral de rang 0 ; et
- à 5,1 ± 0,5 degrés pour l'implant fémoral de rang 8.

Par ailleurs, la présente invention a pour objet une prothèse totale de genou comprenant :
- un implant fémoral ;
- un implant tibial comprenant une portion d'ancrage conformée pour un ancrage dans un tibia ;
- un insert tibial, adapté pour être fixé sur l'implant tibial et conformé pour coopérer avec l'implant fémoral ;
la prothèse totale de genou étant caractérisée en ce que l'implant fémoral est sélectionné dans une gamme d'implants fémoraux selon l'invention.

Les modes de réalisation et les variantes mentionnés ci-avant peuvent être pris isolément ou selon toute combinaison techniquement possible.

La présente invention sera bien comprise et ses avantages ressortiront aussi à la lumière de la description qui va suivre, donnée uniquement à titre d'exemple non limitatif et faite en référence aux dessins annexés, dans lesquels :
- la figure 1 est une vue de côté d'une gamme d'implants fémoraux conforme à l'invention ;
- la figure 2 est une vue de face de la gamme d'implants fémoraux de la figure 1 ;
- la figure 3 est une vue de côté d'un implant fémoral appartenant à la gamme d'implants fémoraux de la figure 1 ;
- la figure 4 est une vue de face de l'implant fémoral de la figure 3 ;
- la figure 5 est une coupe, suivant le plan V à la figure 2; d'une partie de l'implant fémoral de la figure 4 ;
- la figure 6 est une vue en perspective d'une extrémité réséquée d'un fémur à équiper d'un implant fémoral ;
- la figure 7 est une autre vue en perspective d'une extrémité réséquée d'un fémur à équiper d'un implant fémoral ;
- la figure 8 est une vue schématique en coupe de l'implant fémoral de la figure 3 et illustrant des plans de mesure et de construction de la géométrie de l'implant fémoral de la figure 3 ;
- la figure 9 est une vue en perspective d'une extrémité d'un fémur et illustrant l'axe de révolution de condyles postérieurs;
- la figure 10 est une vue de côté d'une extrémité d'un fémur et illustrant l'axe de révolution de condyles postérieurs ;
- la figure 11 est une vue de côté d'une prothèse totale de genou en position d'extension et comprenant un implant fémoral sélectionné dans la gamme d'implants fémoraux de la figure 1 ;
- la figure 12 est une vue similaire à la figure 11 de la prothèse totale de genou de la figure 11 en position de flexion à angle droit.

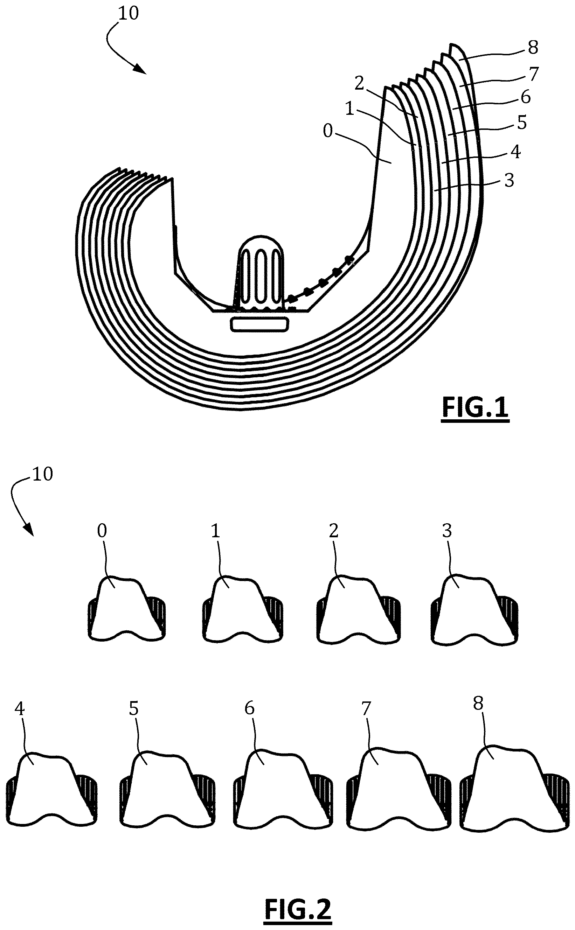

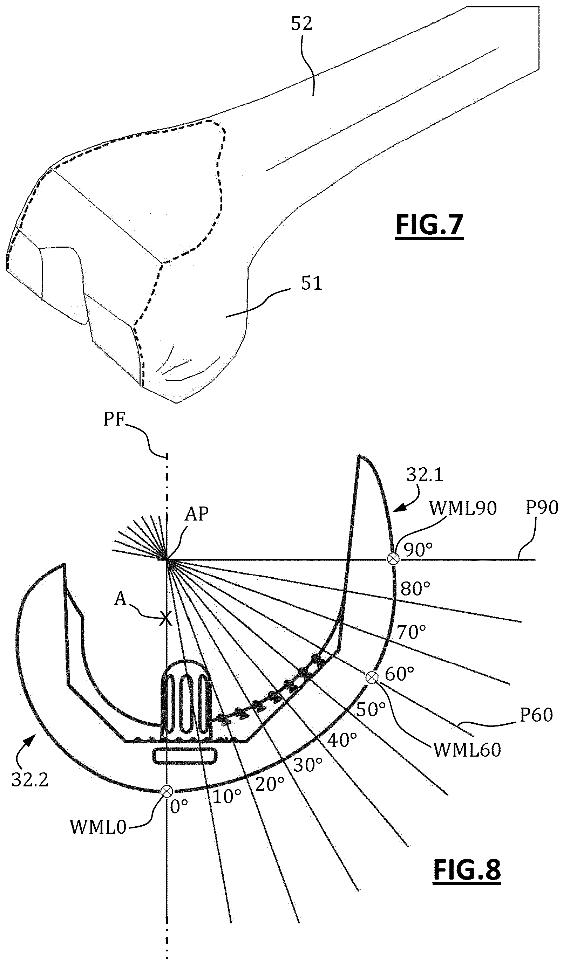

Les figures 1 et 2 illustrent une gamme 10 comprenant au moins sept, en l'occurrence neuf, implants fémoraux 0, 1, 2, 3, 4, 5, 6, 7 et 8 destinés chacun à être implanté sur une extrémité réséquée 51 d'un fémur 52, visible à la figure 7. Les neuf implants fémoraux sont ici ordonnés par dimensions croissantes suivant des rangs allant de 0 à 8.

Chaque implant fémoral 0 à 8 comprend une portion de réception 31 de forme concave et une portion de coopération 32 de forme convexe. Chaque portion de réception 31 définit une cavité adaptée pour recevoir une extrémité réséquée 51 d'un fémur respectif 52. Chaque portion de coopération 32 est agencée pour coopérer avec l'insert tibial.

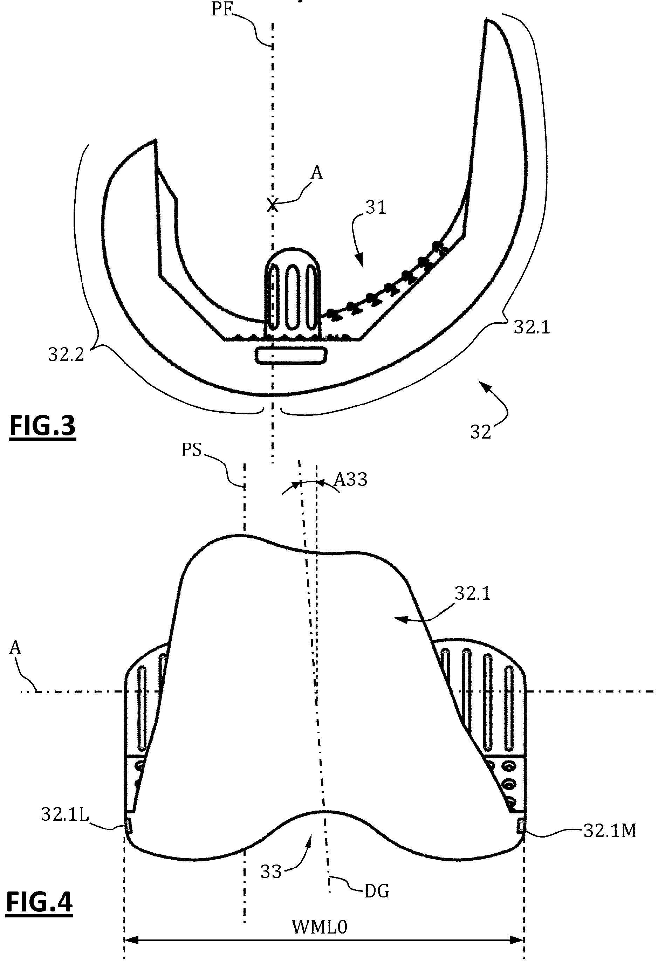

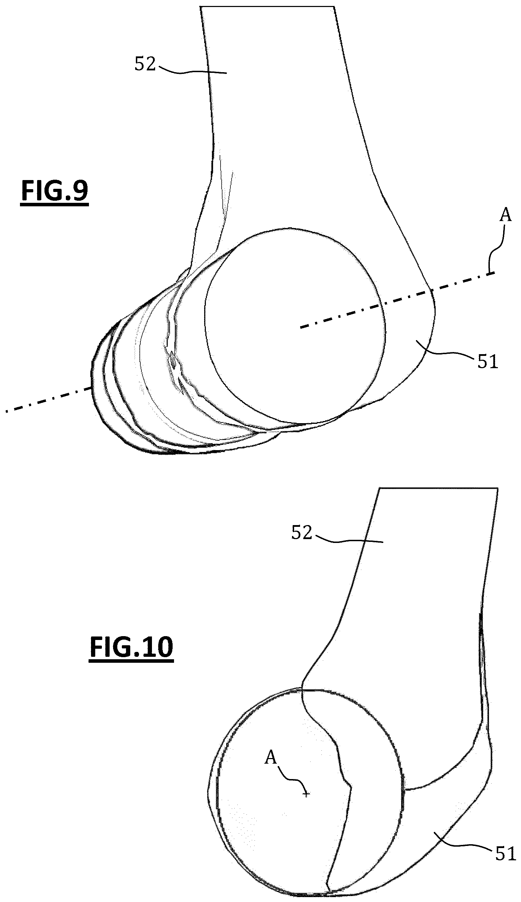

Comme le montre la figure 3, chaque portion de coopération 32 présente une surface antérieure 32.1 et une surface postérieure 32.2. La surface antérieure 32.1 et la surface postérieure 32.2 sont séparées sensiblement par un plan frontal PF, visible aux figures 3 et 8. Le plan frontal PF inclut un axe de révolution de condyles postérieurs A autour duquel tournent les condyles postérieurs (non représentés) lorsque l'implant fémoral 0 à 8 est en service. L'axe de révolution de condyles postérieurs A est visible aux figures 3 et 8 à 10.

Comme le montrent les figures 4 et 8, la surface antérieure respective 32.1 de chaque implant fémoral 0 à 8 présente un bord médial 32.1M et un bord latéral 32.1L. Le bord médial 32.1M et le bord latéral 32.1L délimitent entre eux des largeurs médio-latérales WML0, WML60 etc. qui sont mesurées parallèlement à l'axe de révolution de condyles postérieurs A.

Une largeur médio-latérale primaire WML0 est mesurée dans le plan frontal PF, lequel inclut l'axe de révolution de condyles postérieurs A. Selon le rang de l'implant fémoral 0 à 8, la largeur médio-latérale primaire WML0 est égale :
- à 55,5 ± 1.5 mm, et en particulier égale à 56,0 mm, pour l'implant fémoral de rang 0 ;
- à 58,5 ± 1.5 mm, et en particulier égale à 58,1 mm, pour l'implant fémoral de rang 1 ;
- à 61,5 ± 1.5 mm, et en particulier égale à 60,5 mm, pour l'implant fémoral de rang 2 ;
- à 64,5 ± 1.5 mm, et en particulier égale à 63,1 mm, pour l'implant fémoral de rang 3 ;
- à 67,5 ± 1.5 mm, et en particulier égale à 66,0 mm, pour l'implant fémoral de rang 4 ;
- à 70,5 ± 1.5 mm, et en particulier égale à 69,1 mm, pour l'implant fémoral de rang 5 ;
- à 73,5 ± 1.5 mm, et en particulier égale à 72,4 mm, pour l'implant fémoral de rang 6 ;
- à 76,5 ± 1.5 mm, et en particulier égale à 76,0 mm, pour l'implant fémoral de rang 7 ; et
- à 79,5 ± 1.5 mm, et en particulier égale à 80,0 mm, pour l'implant fémoral de rang 8.

De plus, sur la surface antérieure respective 32.1, le bord médial 32.1M et le bord latéral 32.1L délimitent entre eux une largeur médio-latérale secondaire WML60 qui est mesurée dans un plan secondaire P60 qui inclut un axe proximal AP et qui forme un angle A60 de 60 degrés avec le plan frontal PF incluant l'axe de révolution de condyles postérieurs A en projection dans un plan sagittal PS.

Selon le rang de l'implant fémoral 0 à 8, la largeur médio-latérale secondaire WML60 est égale à :
- à 43,2 ± 1 mm, et en particulier égale à 43,2 mm, pour l'implant fémoral de rang 0 ;
- à 45,0 ± 1 mm, et en particulier égale à 44,9 mm, pour l'implant fémoral de rang 1 ;
- à 47,0 ± 1 mm, et en particulier égale à 47,7 mm, pour l'implant fémoral de rang 2 ;
- à 49,0 ± 1 mm, et en particulier égale à 49,3 mm, pour l'implant fémoral de rang 3 ;
- à 51,0 ± 1 mm, et en particulier égale à 51,4 mm, pour l'implant fémoral de rang 4 ;
- à 53,0 ± 1 mm, et en particulier égale à 53,5 mm, pour l'implant fémoral de rang 5 ;
- à 55,5 ± 1,5 mm, et en particulier égale à 56,7 mm, pour l'implant fémoral de rang 6 ;
- à 58,5 ± 1,5 mm, et en particulier égale à 59,1 mm, pour l'implant fémoral de rang 7 ; et
- à 61,5 ± 1,5 mm, et en particulier égale à 62,2 mm, pour l'implant fémoral de rang 8.

En outre, sur la surface antérieure respective 32.1, le bord médial 32.1M et le bord latéral 32.1L délimitent entre eux une largeur médio-latérale tertiaire WML90 qui est mesurée dans un plan tertiaire P90 qui inclut un axe proximal AP et qui forme un angle A90 de 90 degrés avec le plan frontal PF incluant l'axe de révolution de condyles postérieurs A en projection dans un plan sagittal PS.

Selon le rang de l'implant fémoral 0 à 8, la largeur médio-latérale tertiaire WML90 est égale :
- à 35,0 ± 1 mm, et en particulier égale à 35,7 mm, pour l'implant fémoral de rang 0 ;
- à 37,0 ± 1 mm, et en particulier égale à 37,1 mm, pour l'implant fémoral de rang 1 ;
- à 39,0 ± 1 mm, et en particulier égale à 38,7 mm, pour l'implant fémoral de rang 2 ;
- à 41,0 ± 1 mm, et en particulier égale à 40,6 mm, pour l'implant fémoral de rang 3 ;
- à 43,0 ± 1 mm, et en particulier égale à 42,6 mm, pour l'implant fémoral de rang 4 ;
- à 45,0 ± 1 mm, et en particulier égale à 44,5 mm, pour l'implant fémoral de rang 5 ;
- à 47,0 ± 1 mm, et en particulier égale à 47,1 mm, pour l'implant fémoral de rang 6 ;
- à 49,0 ± 1 mm, et en particulier égale à 48,9 mm, pour l'implant fémoral de rang 7 ; et
- à 51,0 ± 1 mm, et en particulier égale à 51,5 mm, pour l'implant fémoral de rang 8.

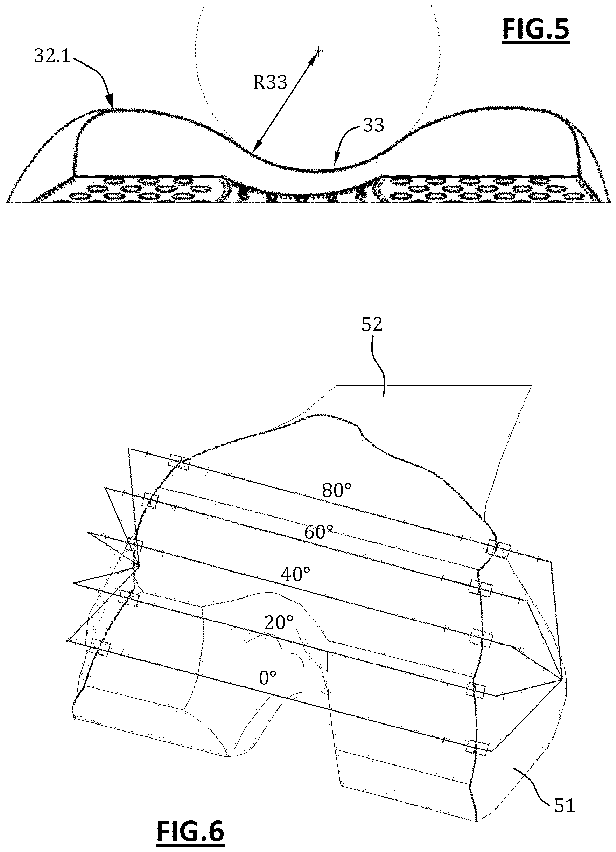

Comme le montrent les figures 4 et 5, la surface antérieure respective 32.1 de chaque implant fémoral 0 à 8 présente une gorge de trochlée 33. La gorge de trochlée 33 a un fond présentant un rayon de courbure R33, qui est mesuré dans le plan frontal PF.

Selon le rang de l'implant fémoral 0 à 8, le rayon de courbure R33 est égal
- à 10,5 ± 0,5 mm, et en particulier égal à 10,8 mm, pour l'implant fémoral de rang 0 ;
- à 11,5 ± 0,5 mm, et en particulier égal à 11,2 mm, pour l'implant fémoral de rang 1 ;
- à 11,5 ± 0,5 mm, et en particulier égal à 11,8 mm, pour l'implant fémoral de rang 2 ;
- à 12,5 ± 0,5 mm, et en particulier égal à 12,4 mm, pour l'implant fémoral de rang 3 ;
- à 13,5 ± 0,5 mm, et en particulier égal à 13,1 mm, pour l'implant fémoral de rang 4 ;
- à 13,5 ± 0,5 mm, et en particulier égal à 13,7 mm, pour l'implant fémoral de rang 5 ;
- à 14,5 ± 0,5 mm, et en particulier égal à 14,3 mm, pour l'implant fémoral de rang 6 ;
- à 14,5 ± 0,5 mm, et en particulier égal à 14,9 mm, pour l'implant fémoral de rang 7 ; et
- à 15,5 ± 0,5 mm, et en particulier égal à 15,7 mm, pour l'implant fémoral de rang 8.

Comme le montre la figure 4, la gorge de trochlée 33 s'étend selon une direction principale DG qui forme un angle A33 avec un plan sagittal PS et en projection dans un plan frontal PF qui est égal à 5 ± 1 degrés pour chaque implant fémoral de rang 0 à 8.

Plus précisément, selon le rang de l'implant fémoral 0 à 8, l'angle A33 est égal à :
- à 5,6 ± 0,5 degrés, et en particulier égal à 5,6 degrés, pour l'implant fémoral de rang 0 ;
- à 5,5 ± 0,5 degrés, et en particulier égal à 5,5 degrés, pour l'implant fémoral de rang 1 ;
- à 5,4 ± 0,5 degrés, et en particulier égal à 5,4 degrés, pour l'implant fémoral de rang 2 ;
- à 5,3 ± 0,5 degrés, et en particulier égal à 5,3 degrés, pour l'implant fémoral de rang 3 ;
- à 5,2 ± 0,5 degrés, et en particulier égal à 5,2 degrés, pour l'implant fémoral de rang 4 ;
- à 5,1 ± 0,5 degrés, et en particulier égal à 5,1 degrés, pour l'implant fémoral de rang 5 ;
- à 5,0 ± 0,5 degrés, et en particulier égal à 5,0 degrés, pour l'implant fémoral de rang 6 ;
- à 4,9 ± 0,5 degrés, et en particulier égal à 4,9 degrés, pour l'implant fémoral de rang 7 ; et
- à 5,1 ± 0,5 degrés, et en particulier égal à 5,1 degrés, pour l'implant fémoral de rang 8.

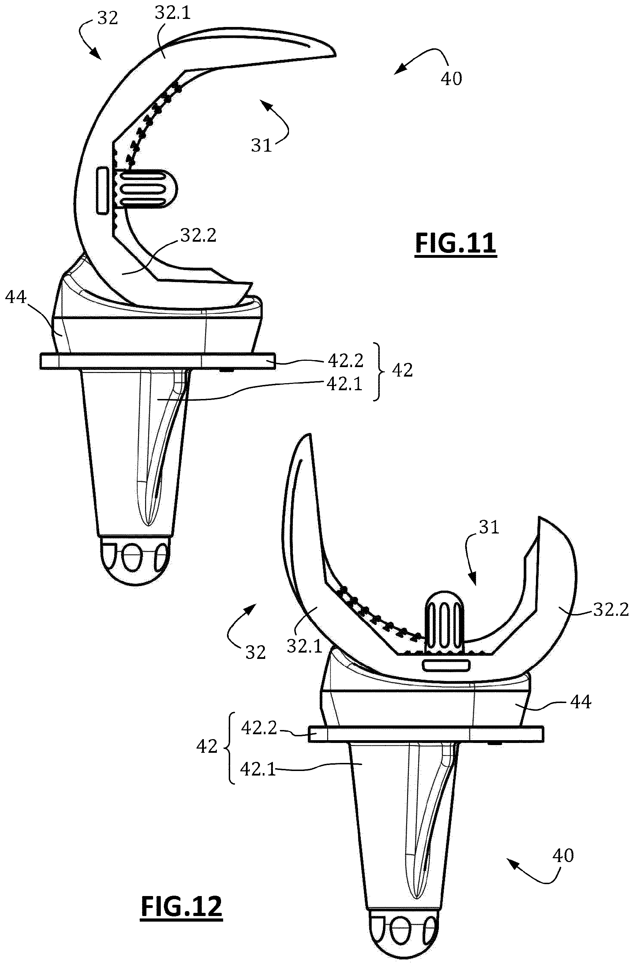

Les figures 11 et 12 illustrent une prothèse totale de genou 40 comprenant l'un des implants fémoraux 0 à 8, en l'occurrence l'implant fémoral 3. La prothèse totale de genou 40 comprend un implant tibial 42 et un insert tibial 44.

L'implant tibial 42 comprend une portion d'ancrage 42.1 qui est conformée pour un ancrage dans un tibia non représenté. L'insert tibial 44 est adapté pour être fixé sur l'implant tibial 42, en l'occurrence sur une plaque de support 42.2. L'insert tibial 44 est conformé pour coopérer avec l'implant fémoral 3. À cet effet, l'insert tibial 44 présente des surfaces congruentes avec des surfaces complémentaires de la portion de coopération 32 de l'implant fémoral..

Pour assembler la prothèse totale de genou 40, on sélectionne l'implant fémoral 3 dans une gamme 10 d'implants fémoraux 0 à 8.

En service, une telle gamme d'implants fémoraux permet au chirurgien de sélectionner un implant fémoral ayant une hauteur suffisante pour réduire les risques de fracture du fémur au-dessus de l'implant fémoral, tout en réduisant les risques que l'implant fémoral ne dépasse sur le côté latéral ou sur le côté médial. Ainsi, une telle gamme d'implants fémoraux permet de conserver l'articulation fémoro-patellaire par les ailerons rotuliens interne et externe, tout en réduisant la largeur médio-latérale maximale de l'implant fémoral.

## Revendications

1. Gamme (10) d'implants fémoraux (0-8), comprenant plusieurs implants fémoraux (0-8) destinés chacun à être implanté sur une extrémité réséquée (51) d'un fémur (52), chaque implant fémoral (0-8) comprenant :
i) une portion de réception (31) définissant une cavité adaptée pour recevoir l'extrémité réséquée (51) du fémur (52), et
ii) une portion de coopération (32) agencée pour coopérer avec un insert tibial, la portion de coopération (32) présentant une surface antérieure (32.1) et une surface postérieure (32.2), la surface antérieure (32.1) et la surface postérieure (32.2) étant séparées sensiblement par un plan frontal (PF) incluant un axe de révolution de condyles postérieurs (A) autour duquel tournent les condyles postérieurs lorsque l'implant fémoral (0-8) est en service,
la surface antérieure respective (32.1) de chaque implant fémoral (0-8) présentant un bord médial (32.1M) et un bord latéral (32.1L) délimitant des largeurs médio-latérales (WML0, WML60) mesurées parallèlement à l'axe de révolution de condyles postérieurs (A),
dans laquelle la gamme (10) d'implants fémoraux (0-8) comprend au moins sept implants fémoraux (1-7) ordonnés par dimensions croissantes suivant des rangs allant de 1 à 7,
et dans laquelle une largeur médio-latérale primaire (WML0), mesurée dans le plan frontal (PF) incluant l'axe de révolution de condyles postérieurs (A), est égale :
- à 58,5 ± 1.5 mm pour l'implant fémoral de rang 1 ;
- à 61,5 ± 1.5 mm pour l'implant fémoral de rang 2 ;
- à 64,5 ± 1.5 mm pour l'implant fémoral de rang 3 ;
- à 67,5 ± 1.5 mm pour l'implant fémoral de rang 4 ;
- à 70,5 ± 1.5 mm pour l'implant fémoral de rang 5 ;
- à 73,5 ± 1.5 mm pour l'implant fémoral de rang 6 ; et
- à 76,5 ± 1.5 mm pour l'implant fémoral de rang 7 ;
la gamme (10) d'implants fémoraux (0-8) étant **caractérisée en ce que** la surface antérieure respective (32.1) de chaque implant fémoral (0-8) présente une largeur médio-latérale secondaire (WML60), mesurée dans un plan secondaire (P60) qui inclut un axe proximal (AP) et qui forme un angle (A60) de 60 degrés avec le plan frontal (PF) incluant l'axe de révolution de condyles postérieurs (A) en projection dans un plan sagittal (PS), égale :
- à 45,0 ± 1 mm pour l'implant fémoral de rang 1 ;
- à 47,0 ± 1 mm pour l'implant fémoral de rang 2 ;
- à 49,0 ± 1 mm pour l'implant fémoral de rang 3 ;
- à 51,0 ± 1 mm pour l'implant fémoral de rang 4 ;
- à 53,0 ± 1 mm pour l'implant fémoral de rang 5 ;
- à 55,5 ± 1,5 mm pour l'implant fémoral de rang 6 ; et
- à 58,5 ± 1,5 mm pour l'implant fémoral de rang 7.

2. Gamme (10) d'implants fémoraux (0-8) selon la revendication 1, dans laquelle la largeur médio-latérale primaire (WML0) est égale :
- à 58,1 mm pour l'implant fémoral de rang 1 ;
- à 60,5 mm pour l'implant fémoral de rang 2 ;
- à 63,1 mm pour l'implant fémoral de rang 3 ;
- à 66,0 mm pour l'implant fémoral de rang 4 ;
- à 69,1 mm pour l'implant fémoral de rang 5 ;
- à 72,4 mm pour l'implant fémoral de rang 6 ;
- à 76,0 mm pour l'implant fémoral de rang 7.

3. Gamme (10) d'implants fémoraux (0-8) selon l'une quelconque des revendications 1 et 2, dans laquelle la largeur médio-latérale secondaire (WML60) est égale :
- à 44,9 mm pour l'implant fémoral de rang 1 ;
- à 47,7 mm pour l'implant fémoral de rang 2 ;
- à 49,3 mm pour l'implant fémoral de rang 3 ;
- à 51,4 mm pour l'implant fémoral de rang 4 ;
- à 53,5 mm pour l'implant fémoral de rang 5 ;
- à 56,7 mm pour l'implant fémoral de rang 6 ; et
- à 59,1 mm pour l'implant fémoral de rang 7.

4. Gamme (10) d'implants fémoraux (0-8) selon l'une quelconque des revendications 1 à 3, comprenant au moins neuf implants fémoraux (0-8) ordonnés par dimensions croissantes suivant des rangs allant de 0 à 8,
et dans laquelle la largeur médio-latérale primaire (WML0) est égale :
- à 55,5 ± 1.5 mm pour l'implant fémoral de rang 0 ; et
- à 79,5 ± 1.5 mm pour l'implant fémoral de rang 8
et dans laquelle la largeur médio-latérale secondaire (WML60) est égale :
- à 43,2 ± 1 mm pour l'implant fémoral de rang 0 ; et
- à 61,5 ± 1,5 mm pour l'implant fémoral de rang 8.

5. Gamme (10) d'implants fémoraux (0-8) selon la revendication 4, dans laquelle la largeur médio-latérale primaire (WML0) est égale :
- à 56,0 mm pour l'implant fémoral de rang 0 ; et
- à 80,0 mm pour l'implant fémoral de rang 8.

6. Gamme (10) d'implants fémoraux (0-8) selon l'une quelconque des revendications 4 et 5, dans laquelle la largeur médio-latérale secondaire (WML60) est égale :
- à 43,2 mm pour l'implant fémoral de rang 0 ; et
- à 62,2 mm pour l'implant fémoral de rang 8.

7. Gamme (10) d'implants fémoraux (0-8) selon l'une quelconque des revendications précédentes, dans laquelle la surface antérieure respective (32.1) de chaque implant fémoral (0-8) présente une largeur médio-latérale tertiaire (WML90), mesurée dans un plan tertiaire (P90) qui inclut un axe proximal (AP) et qui forme un angle (A90) de 90 degrés avec le plan frontal (PF) incluant l'axe de révolution de condyles postérieurs (A) en projection dans un plan sagittal (PS), égale :
- à 37,0 ± 1 mm pour l'implant fémoral de rang 1 ;
- à 39,0 ± 1 mm pour l'implant fémoral de rang 2 ;
- à 41,0 ± 1 mm pour l'implant fémoral de rang 3 ;
- à 43,0 ± 1 mm pour l'implant fémoral de rang 4 ;
- à 45,0 ± 1 mm pour l'implant fémoral de rang 5 ;
- à 47,0 ± 1 mm pour l'implant fémoral de rang 6 ; et
- à 49,0 ± 1 mm pour l'implant fémoral de rang 7.

8. Gamme (10) d'implants fémoraux (0-8) selon la revendication 7, dans laquelle la largeur médio-latérale (WML90) est égale :
- à 37,1 mm pour l'implant fémoral de rang 1 ;
- à 38,7 mm pour l'implant fémoral de rang 2 ;
- à 40,6 mm pour l'implant fémoral de rang 3 ;
- à 42,6 mm pour l'implant fémoral de rang 4 ;
- à 44,5 mm pour l'implant fémoral de rang 5 ;
- à 47,1 mm pour l'implant fémoral de rang 6 ; et
- à 48,9 mm pour l'implant fémoral de rang 7.

9. Gamme (10) d'implants fémoraux (0-8) selon les revendications 4 et 7, dans laquelle la largeur médio-latérale tertiaire (WML90) est égale :
- à 35,0 ± 1 mm pour l'implant fémoral de rang 0 ; et
- à 51,0 ± 1 mm pour l'implant fémoral de rang 8

10. Gamme (10) d'implants fémoraux (0-8) selon l'une quelconque des revendications précédentes, dans laquelle la surface antérieure respective (32.1) de chaque implant fémoral (0-8) présente une gorge de trochlée (33), la gorge de trochlée (33) ayant un fond présentant un rayon de courbure (R33), mesuré dans le plan frontal (PF) incluant l'axe de révolution de condyles postérieurs (A), égal :
- à 11,5 ± 0,5 mm pour l'implant fémoral de rang 1 ;
- à 11,5 ± 0,5 mm pour l'implant fémoral de rang 2 ;
- à 12,5 ± 0,5 mm pour l'implant fémoral de rang 3 ;
- à 13,5 ± 0,5 mm pour l'implant fémoral de rang 4 ;
- à 13,5 ± 0,5 mm pour l'implant fémoral de rang 5 ;
- à 14,5 ± 0,5 mm pour l'implant fémoral de rang 6 ; et
- à 14,5 ± 0,5 mm pour l'implant fémoral de rang 7.

11. Gamme (10) d'implants fémoraux (0-8) selon les revendications 4 et 10, dans laquelle le fond de la gorge de trochlée (33) présente un rayon de courbure (R33) égal :
- à 10,5 ± 0,5 mm pour l'implant fémoral de rang 0 ; et
- à 15,5 ± 0,5 mm pour l'implant fémoral de rang 8.

12. Gamme (10) d'implants fémoraux (0-8) selon l'une quelconque des revendications 10 et 11, dans laquelle la gorge de trochlée (33) forme, avec un plan sagittal (PS) et en projection dans un plan frontal (PF), un angle (A33) égal à 5 degrés ± 1 degré pour chaque implant fémoral de la gamme (10).

13. Gamme (10) d'implants fémoraux (0-8) selon la revendication 12, dans laquelle la gorge de trochlée (33) forme, avec le plan sagittal (PS) et en projection dans le plan frontal (PF), un angle (A33) égal :
- à 5,5 ± 0,5 degrés pour l'implant fémoral de rang 1 ;
- à 5,4 ± 0,5 degrés pour l'implant fémoral de rang 2 ;
- à 5,3 ± 0,5 degrés pour l'implant fémoral de rang 3 ;
- à 5,2 ± 0,5 degrés pour l'implant fémoral de rang 4 ;
- à 5,1 ± 0,5 degrés pour l'implant fémoral de rang 5 ;
- à 5,0 ± 0,5 degrés pour l'implant fémoral de rang 6 ; et
- à 4,9 ± 0,5 degrés pour l'implant fémoral de rang 7.

14. Gamme (10) d'implants fémoraux (0-8) selon les revendications 4 et 13, dans laquelle la gorge de trochlée (33) forme, avec le plan sagittal (PS) et en projection dans le plan frontal (PF), un angle (A33) égal :
- à 5,6 ± 0,5 degrés pour l'implant fémoral de rang 0 ; et
- à 5,1 ± 0,5 degrés pour l'implant fémoral de rang 8.

## Patentansprüche

1. Sortiment (10) von Femurimplantaten (0-8), umfassend mehrere Femurimplantate (0-8), die dazu vorgesehen sind, jeweils an einem resezierten Ende (51) eines Femurs (52) implantiert zu werden, wobei jedes Femurimplantat (0-8) umfasst:
i) einen Aufnahmeabschnitt (31), der eine Vertiefung definiert, die zum Aufnehmen des resezierten Endes (51) des Femurs (52) angepasst ist, und
ii) einen Zusammenwirkungsabschnitt (32), der zum Zusammenwirken mit einem Tibiaeinsatz eingerichtet ist, wobei der Zusammenwirkungsabschnitt (32) eine vordere Fläche (32.1) und eine hintere Fläche (32.2) aufweist, wobei die vordere Fläche (32.1) und die hintere Fläche (32.2) im Wesentlichen durch eine Frontalebene (PF) getrennt sind, die eine Drehachse von hinteren Kondylen (A) beinhaltet, um die sich die hinteren Kondylen drehen, wenn das Femurimplantat (0-8) im Einsatz ist,
wobei die jeweilige vordere Fläche (32.1) jedes Femurimplantats (0-8) einen medialen Rand (32.1M) und einen lateralen Rand (32.1L) aufweist, die mediolaterale Breiten (WML0, WML60) abgrenzen, die parallel zur Drehachse von hinteren Kondylen (A) gemessen werden,
wobei das Sortiment (10) von Femurimplantaten (0-8) mindestens sieben Femurimplantate (1-7) umfasst, die anhand von zunehmenden Abmessungen gemäß Rangordnungen, die von 1 bis 7 reichen, geordnet sind,
und wobei eine primäre mediolaterale Breite (WML0), die in der Frontalebene (PF), die die Drehachse von hinteren Kondylen (A) beinhaltet, gemessen wird, gleich:
- 58,5 ± 1,5 mm für das Femurimplantat der Rangordnung 1;
- 61,5 ± 1,5 mm für das Femurimplantat der Rangordnung 2;
- 64,5 ± 1,5 mm für das Femurimplantat der Rangordnung 3;
- 67,5 ± 1,5 mm für das Femurimplantat der Rangordnung 4;
- 70,5 ± 1,5 mm für das Femurimplantat der Rangordnung 5;
- 73,5 ± 1,5 mm für das Femurimplantat der Rangordnung 6; und
- 76,5 ± 1,5 mm für das Femurimplantat der Rangordnung 7 ist;
wobei das Sortiment (10) von Femurimplantaten (0-8) **dadurch gekennzeichnet ist, dass** die jeweilige vordere Fläche (32.1) jedes Femurimplantats (0-8) eine sekundäre mediolaterale Breite (WML60), die in einer sekundären Ebene (P60), die eine proximale Achse (AP) beinhaltet und die einen Winkel (A60) von 60 Grad mit der Frontalebene (PF), die die Drehachse von hinteren Kondylen (A) beinhaltet, bildet, projiziert in eine Sagittalebene (PS), gemessen wird, aufweist, die gleich:
- 45,0 ± 1 mm für das Femurimplantat der Rangordnung 1;
- 47,0 ± 1 mm für das Femurimplantat der Rangordnung 2;
- 49,0 ± 1 mm für das Femurimplantat der Rangordnung 3;
- 51,0 ± 1 mm für das Femurimplantat der Rangordnung 4;
- 53,0 ± 1 mm für das Femurimplantat der Rangordnung 5;
- 55,5 ± 1,5 mm für das Femurimplantat der Rangordnung 6; und
- 58,5 ± 1,5 mm für das Femurimplantat der Rangordnung 7 ist.

2. Sortiment (10) von Femurimplantaten (0-8) nach Anspruch 1, wobei die primäre mediolaterale Breite (WML0) gleich:
- 58,1 mm für das Femurimplantat der Rangordnung 1;
- 60,5 mm für das Femurimplantat der Rangordnung 2;
- 63,1 mm für das Femurimplantat der Rangordnung 3;
- 66,0 mm für das Femurimplantat der Rangordnung 4;
- 69,1 mm für das Femurimplantat der Rangordnung 5;
- 72,4 mm für das Femurimplantat der Rangordnung 6;
- 76,0 mm für das Femurimplantat der Rangordnung 7 ist.

3. Sortiment (10) von Femurimplantaten (0-8) nach einem der Ansprüche 1 und 2, wobei die sekundäre mediolaterale Breite (WML60) gleich:
- 44,9 mm für das Femurimplantat der Rangordnung 1;
- 47,7 mm für das Femurimplantat der Rangordnung 2;
- 49,3 mm für das Femurimplantat der Rangordnung 3;
- 51,4 mm für das Femurimplantat der Rangordnung 4;
- 53,5 mm für das Femurimplantat der Rangordnung 5;
- 56,7 mm für das Femurimplantat der Rangordnung 6; und
- 59,1 mm für das Femurimplantat der Rangordnung 7 ist.

4. Sortiment (10) von Femurimplantaten (0-8) nach einem der Ansprüche 1 bis 3, umfassend mindestens neun Femurimplantate (0-8), die anhand von zunehmenden Abmessungen gemäß Rangordnungen, die von 0 bis 8 reichen, geordnet sind,
und wobei die primäre mediolaterale Breite (WML0) gleich:
- 55,5 ± 1,5 mm für das Femurimplantat der Rangordnung 0; und
- 79,5 ± 1,5 mm für das Femurimplantat der Rangordnung 8 ist,
und wobei die sekundäre mediolaterale Breite (WML60) gleich:
- 43,2 ± 1 mm für das Femurimplantat der Rangordnung 0; und
- 61,5 ± 1,5 mm für das Femurimplantat der Rangordnung 8 ist.

5. Sortiment (10) von Femurimplantaten (0-8) nach Anspruch 4, wobei die primäre mediolaterale Breite (WML0) gleich:
- 56,0 mm für das Femurimplantat der Rangordnung 0; und
- 80,0 mm für das Femurimplantat der Rangordnung 8 ist.

6. Sortiment (10) von Femurimplantaten (0-8) nach einem der Ansprüche 4 und 5, wobei die sekundäre mediolaterale Breite (WML60) gleich:
- 43,2 mm für das Femurimplantat der Rangordnung 0; und
- 62,2 mm für das Femurimplantat der Rangordnung 8 ist.

7. Sortiment (10) von Femurimplantaten (0-8) nach einem der vorhergehenden Ansprüche, wobei die jeweilige vordere Fläche (32.1) jedes Femurimplantats (0-8) eine tertiäre mediolaterale Breite (WML90), die in einer tertiären Ebene (P90), die eine proximale Achse (AP) beinhaltet und die einen Winkel (A90) von 90 Grad mit der Frontalebene (PF), die die Drehachse von hinteren Kondylen (A) beinhaltet, bildet, projiziert in eine Sagittalebene (PS), gemessen wird, aufweist, die gleich:
- 37,0 ± 1 mm für das Femurimplantat der Rangordnung 1;
- 39,0 ± 1 mm für das Femurimplantat der Rangordnung 2;
- 41,0 ± 1 mm für das Femurimplantat der Rangordnung 3;
- 43,0 ± 1 mm für das Femurimplantat der Rangordnung 4;
- 45,0 ± 1 mm für das Femurimplantat der Rangordnung 5;
- 47,0 ± 1 mm für das Femurimplantat der Rangordnung 6; und
- 49,0 ± 1 mm für das Femurimplantat der Rangordnung 7 ist.

8. Sortiment (10) von Femurimplantaten (0-8) nach Anspruch 7, wobei die tertiäre mediolaterale Breite (WML90) gleich:
- 37,1 mm für das Femurimplantat der Rangordnung 1;
- 38,7 mm für das Femurimplantat der Rangordnung 2;
- 40,6 mm für das Femurimplantat der Rangordnung 3;
- 42,6 mm für das Femurimplantat der Rangordnung 4;
- 44,5 mm für das Femurimplantat der Rangordnung 5;
- 47,1 mm für das Femurimplantat der Rangordnung 6; und
- 48,9 mm für das Femurimplantat der Rangordnung 7 ist.

9. Sortiment (10) von Femurimplantaten (0-8) nach einem der Ansprüche 4 und 7, wobei die tertiäre mediolaterale Breite (WML90) gleich:
- 35,0 ± 1 mm für das Femurimplantat der Rangordnung 0; und
- 51,0 ± 1 mm für das Femurimplantat der Rangordnung 8 ist.

10. Sortiment (10) von Femurimplantaten (0-8) nach einem der vorhergehenden Ansprüche, wobei die jeweilige vordere Fläche (32.1) jedes Femurimplantats (0-8) eine Trochleakehle (33) aufweist, wobei die Trochleakehle (33) einen Boden hat, der einen Krümmungsradius (R33), der in der Frontalebene (PF), die die Drehachse von hinteren Kondylen (A) beinhaltet, gemessen wird, aufweist, der gleich:
- 11,5 ± 0,5 mm für das Femurimplantat der Rangordnung 1;
- 11,5 ± 0,5 mm für das Femurimplantat der Rangordnung 2;
- 12,5 ± 0,5 mm für das Femurimplantat der Rangordnung 3;
- 13,5 ± 0,5 mm für das Femurimplantat der Rangordnung 4;
- 13,5 ± 0,5 mm für das Femurimplantat der Rangordnung 5;
- 14,5 ± 0,5 mm für das Femurimplantat der Rangordnung 6; und
- 14,5 ± 0,5 mm für das Femurimplantat der Rangordnung 7 ist.

11. Sortiment (10) von Femurimplantaten (0-8) nach einem der Ansprüche 4 und 10, wobei der Boden der Trochleakehle (33) einen Krümmungsradius (R33) aufweist, der gleich:
- 10,5 ± 0,5 mm für das Femurimplantat der Rangordnung 0; und
- 15,5 ± 0,5 mm für das Femurimplantat der Rangordnung 8 ist.

12. Sortiment (10) von Femurimplantaten (0-8) nach einem der Ansprüche 10 und 11, wobei die Trochleakehle (33) mit einer Sagittalebene (PS) und projiziert in eine Frontalebene (PF) einen Winkel (A33) bildet, der gleich 5 Grad ± 1 Grad für jedes Femurimplantat des Sortiments (10) ist.

13. Sortiment (10) von Femurimplantaten (0-8) nach Anspruch 12, wobei die Trochleakehle (33) mit der Sagittalebene (PS) und projiziert in die Frontalebene (PF) einen Winkel (A33) bildet, der gleich:
- 5,5 ± 0,5 Grad für das Femurimplantat der Rangordnung 1;
- 5,4 ± 0,5 Grad für das Femurimplantat der Rangordnung 2;
- 5,3 ± 0,5 Grad für das Femurimplantat der Rangordnung 3;
- 5,2 ± 0,5 Grad für das Femurimplantat der Rangordnung 4;
- 5,1 ± 0,5 Grad für das Femurimplantat der Rangordnung 5;
- 5,0 ± 0,5 Grad für das Femurimplantat der Rangordnung 6; und
- 4,9 ± 0,5 Grad für das Femurimplantat der Rangordnung 7 ist.

14. Sortiment (10) von Femurimplantaten (0-8) nach den Ansprüchen 4 und 13, wobei die Trochleakehle (33) mit der Sagittalebene (PS) und projiziert in die Frontalebene (PF) einen Winkel (A33) bildet, der gleich:
- 5,6 ± 0,5 Grad für das Femurimplantat der Rangordnung 0; und
- 5,1 ± 0,5 Grad für das Femurimplantat der Rangordnung 8 ist.

## Claims

1. A range (10) of femoral implants (0-8), comprising a plurality of femoral implants (0-8) each intended to be implanted on a resected end (51) of a femur (52), each femoral implant (0-8) comprising:
i. a receiving portion (31) defining a cavity suitable for receiving the resected end (51) of the femur (52), and
ii. a cooperating portion (32) arranged to cooperate with a tibial insert, the cooperating portion (32) having an anterior surface (32.1) and a posterior surface (32.2), the anterior surface (32.1) and the posterior surface (32.2) being separated substantially by a frontal plane (PF) including an axis of revolution of the posterior condyles (A) about which the posterior condyles rotate when the femoral implant (0-8) is in use,
the respective anterior surface (32.1) of each femoral implant (0-8) having a medial edge (32.1M) and a lateral edge (32.1 L) delimiting medio-lateral widths (WML0, WML60) measured parallel to the axis of revolution of the posterior condyles (A),
wherein the range (10) of femoral implants (0-8) comprises at least seven femoral implants (1-7) ordered by increasing dimensions according to rows ranging from 1 to 7,
and wherein a primary medio-lateral width (WML0), measured in the frontal plane (PF) including the axis of revolution of the posterior condyles (A), is equal to:
- 58.5 ±1.5 mm for the femoral implant of row 1;
- 61.5 ±1.5 mm for the femoral implant of row 2;
- 64.5 ±1.5 mm for the femoral implant of row 3;
- 67.5 ±1.5 mm for the femoral implant of row 4;
- 70.5 ±1.5 mm for the femoral implant of row 5;
- 73.5 ±1.5 mm for the femoral implant of row 6; and
- 76.5 ±1.5 mm for the femoral implant of row 7;
the range (10) of femoral implants (0-8) being **characterized in that** the respective anterior surface (32.1) of each femoral implant (0-8) has a secondary medio-lateral width (WML60), measured in a secondary plane (P60) which includes a proximal axis (AP) and which forms an angle (A60) of 60 degrees with the frontal plane (PF) including the axis of revolution of the posterior condyles (A) in projection in a sagittal plane (PS), equal to:
- 45.0 ±1 mm for the femoral implant of row 1;
- 47.0 ±1 mm for the femoral implant of row 2;
- 49.0 ±1 mm for the femoral implant of row 3;
- 51.0 ±1 mm for the femoral implant of row 4;
- 53.0 ±1 mm for the femoral implant of row 5;
- 55.5 ±1.5 mm for the femoral implant of row 6; and
- 58.5 ±1.5 mm for the femoral implant of row 7.

2. The range (10) of femoral implants (0-8) according to claim 1, wherein the primary medio-lateral width (WML0) is equal to:
- 58.1 mm for the femoral implant of row 1;
- 60.5 mm for the femoral implant of row 2;
- 63.1 mm for the femoral implant of row 3;
- 66.0 mm for the femoral implant of row 4;
- 69.1 mm for the femoral implant of row 5;
- 72.4 mm for the femoral implant of row 6; and
- 76.0 mm for the femoral implant of row 7.

3. The range (10) of femoral implants (0-8) according to any one of claims 1 and 2, wherein the secondary medio-lateral width (WML60) is equal to:
- 44.9 mm for the femoral implant of row 1;
- 47.7 mm for the femoral implant of row 2;
- 49.3 mm for the femoral implant of row 3;
- 51.4 mm for the femoral implant of row 4;
- 53.5 mm for the femoral implant of row 5;
- 56.7 mm for the femoral implant of row 6; and
- 59.1 mm for the femoral implant of row 7.

4. The range (10) of femoral implants (0-8) according to any one of claims 1 to 3, comprising at least nine femoral implants (0-8) ordered by increasing dimensions according to rows ranging from 0 to 8,
and wherein the primary medio-lateral width (WML0) is equal to:
- 55.5 ±1.5 mm for the femoral implant of row 0; and
- 79.5 ±1.5 mm for the femoral implant of row 8;
and wherein the secondary medio-lateral width (WML60) is equal to:
- 43.2 ±1 mm for the femoral implant of row 0; and
- 61.5 ±1.5 mm for the femoral implant of row 8.

5. The range (10) of femoral implants (0-8) according to claim 4, wherein the primary medio-lateral width (WML0) is equal to:
- 56.0 mm for the femoral implant of row 0; and
- 80.0 mm for the femoral implant of row 8.

6. The range (10) of femoral implants (0-8) according to any one of claims 4 to 5, wherein the secondary medio-lateral width (WML60) is equal to:
- 43.2 mm for the femoral implant of row 0; and
- 62.2 mm for the femoral implant of row 8.

7. The range (10) of femoral implants (0-8) according to any one of the preceding claims, wherein the respective anterior surface (32.1) of each femoral implant (0-8) has a tertiary medio-lateral width (WML90), measured in a tertiary plane (P90) which includes a proximal axis (AP) and forms an angle (A90) of 90 degrees with the frontal plane (PF) including the axis of revolution of the posterior condyles (A) in projection in a sagittal plane (PS), equal to:
- 37.0 ±1 mm for the femoral implant of row 1;
- 39.0 ±1 mm for the femoral implant of row 2;
- 41.0 ±1 mm for the femoral implant of row 3;
- 43.0 ±1 mm for the femoral implant of row 4;
- 45.0 ±1 mm for the femoral implant of row 5;
- 47.0 ±1 mm for the femoral implant of row 6; and
- 49.0 ±1 mm for the femoral implant of row 7.

8. The range (10) of femoral implants (0-8) according to claim 7, wherein the medio-lateral width (WML90) is equal to:
- 37.1 mm for the femoral implant of row 1;
- 38.7 mm for the femoral implant of row 2;
- 40.6 mm for the femoral implant of row 3;
- 42.6 mm for the femoral implant of row 4;
- 44.5 mm for the femoral implant of row 5;
- 47.1 mm for the femoral implant of row 6; and
- 48.9 mm for the femoral implant of row 7.

9. The range (10) of femoral implants (0-8) according to claims 4 and 7, wherein the tertiary medio-lateral width (WML90) is equal to:
- 35.0 ±1 mm for the femoral implant of row 0; and
- 51.0 ±1 mm for the femoral implant of row 8.

10. The range (10) of femoral implants (0-8) according to any one of the preceding claims, wherein the respective anterior surface (32.1) of each femoral implant (0-8) has a trochlea groove (33), the trochlea groove (33) having a base comprising a radius of curvature (R33), measured in the frontal plane (PF) including the axis of revolution of the posterior condyles (A), equal to:
- 11.5 ±0.5 mm for the femoral implant of row 1;
- 11.5 ±0.5 mm for the femoral implant of row 2;
- 12.5 ±0.5 mm for the femoral implant of row 3;
- 13.5 ±0.5 mm for the femoral implant of row 4;
- 13.5 ±0.5 mm for the femoral implant of row 5;
- 14.5 ±0.5 mm for the femoral implant of row 6; and
- 14.5 ±0.5 mm for the femoral implant of row 7.

11. The range (10) of femoral implants (0-8) according to claims 4 and 10, wherein the base of the trochlea groove (33) has a radius of curvature (R33) equal to:
- 10.5 ±0.5 mm for the femoral implant of row 0; and
- 15.5 ±0.5 mm for the femoral implant of row 8.

12. The range (10) of femoral implants (0-8) according to any one of claims 10 and 11, wherein the trochlea groove (33) forms, with a sagittal plane (PS) and in projection in a frontal plane (PF), an angle (A33) equal to 5 degrees ±1 degree for each femoral implant of the range (10).

13. The range (10) of femoral implants (0-8) according to claim 12, wherein the trochlea groove (33) forms with the sagittal plane (PS) and in projection in the frontal plane (PF), an angle (A33) equal to:
- 5.5 ±0.5 degrees for the femoral implant of row 1;
- 5.4 ±0.5 degrees for the femoral implant of row 2;
- 5.3 ±0.5 degrees for the femoral implant of row 3;
- 5.2 ±0.5 degrees for the femoral implant of row 4;
- 5.1 ±0.5 degrees for the femoral implant of row 5;
- 5.0 ±0.5 degrees for the femoral implant of row 6; and
- 4.9 ±0.5 degrees for the femoral implant of row 7.

14. The range (10) of femoral implants (0-8) according to claims 4 and 13, wherein the trochlea groove (33) forms with the sagittal plane (PS) and in projection in the frontal plane (PF), an angle (A33) equal to:
- 5.6 ±0.5 degrees for the femoral implant of row 0; and
- 5.1 ±0.5 degrees for the femoral implant of row 8.
